# EUROPEAN PATENT SPECIFICATION

(11) **EP 1 087 750 B1**
(45) Date of publication and mention of the grant of the patent: **12.11.2003**
(21) Application number: 99928458.1
(22) Date of filing: 08.06.1999
(51) Int. Cl.: A61K 9/12, A61K 31/135, A61K 31/46

(54) **PHARMACEUTICAL FORMULATIONS FOR AEROSOLS WITH TWO OR MORE ACTIVE SUBSTANCES**
ZWEI ODER MEHRERE WIRKSTOFFE ENTHALTENDE PHARMAZEUTISCHE AEROSOLFORMULIERUNGEN
PREPARATIONS PHARMACEUTIQUES POUR AEROSOLS A DEUX PRINCIPES ACTIFS OU PLUS

(30) Priority: 18.06.1998 DE 19827178; 19.09.1998 DE 19842963
(43) Date of publication of application: 04.04.2001
(73) Proprietor: BOEHRINGER INGELHEIM PHARMACEUTICALS INC., Ridgefield, Connecticut 06877-0368 (US)
(72) Inventor: McNAMARA, Daniel, P., Waterbury, CT 06708 (US); DeSTEFANO, George, A., Brookfield, CT 06804 (US)
(74) Representative: Laudien, Dieter, Dr.
(86) International application number: US9912785
(87) International publication number: WO99065464

(56) References cited:
- EP-A- 0 499 344
- WO-A-94/13262
- WO-A-98/01147
- US-A- 5 589 156
- US-A- 5 603 918

## Description

The present invention relates to new pharmaceutical formulations for aerosols with at least two or more active substances for use by inhalation or by the nasal route.

### State of the art

In propellant-driven metered dose inhalers (MDI) the active substances can be formulated as solutions or suspensions. The vast majority of aerosol formulations for MDI's are prepared as suspensions, especially if the preparation contains more than one active substance. Formulations in the form of solutions are used only to a limited extent. In these cases, the formulations normally contain only one active substance.

WO-A-98 01147 discloses a solution formulation of Cyclosporin A in 1,1,1,2,3,3,3-heptafluoropropane which is suitable for administration to a patient by inhalation using any standard medicinal aerosol device.

US-A-5 589 156 discloses aerosol compositions used for anesthetizing mammals in both human and veterinary applications, said compositions including prilocaine base solubilized in the hydrofluorocarbon (HFC) propellants 1,1,1,2-tetrafluorethane and 1,1,1,2,3,3,3-heptafluoropropane.

US-A-5 603 918 discloses an aerosol formulation which contains an effective amount of a pharmaceutically acceptable salt of ipratropium and an effective amount of a pharmaceutically acceptable salt of albuterol in combination with an effective amount of soya lecithin as a suspending agent and a propellant.

As a rule, in a suspension, the chemical stability of the active substances is noticeably higher than in a solution. Additionally, in a suspension the active substance can be more highly concentrated than in a solution, with the result that suspension type formulation enable higher doses to be administered.

A major disadvantage of suspension-formulations is the fact that over time (e.g. during storage) the suspended particles clump together to form bigger, more or less stable agglomerates or form loose flakes, sediments or floating layers, or in the worst case, particle growth, which significantly impairs the pharmaceutical quality of the product. The size of the particles formed or the speed of particle growth are influenced by the solubility features of the liquid phase. Thus, ingress of humidity during storage or a desired increase in polarity, e.g. achieved by adding co-solvents, can have a devastating effect on the quality of the medical end product, particularly if the suspended particles have polar structure elements. The suspension can be physically stabilised by the addition of surfactants, by reducing the harmful effects of moisture and/or particle growth so that suspended particles can be held in suspension for longer.

Natural solution-type formulations are not affected by the problems of increasing particle size or de-mixing processes such as sedimentation or flocculation. However, in this case there is a serious risk of chemical degradation. A further disadvantage is the fact that the limited solubility of the ingredients can prevent administration in high doses. In the past, the chlorofluorohydrocarbons TG 11 (trichlorofluoromethane), TG 12 (dichlorodifluoromethane) and TG 114 (dichlorotetrafluoroethane) have proved particularly suitable as solvents. The solubility of the ingredients can be increased by the addition of co-solvents. In addition, it is usually necessary to take additional measures to chemically stabilise the dissolved components.

Up till now, CFCs such as the above-mentioned TG 11, for example, have often been used as propellants. However, since CFCs have been linked with the destruction of the ozone layer, their manufacture and use are being phased out. The intention is to replace them with special fluorohydrocarbons (HFC) which are less destructive to the ozone layer but have completely different solubility features. The toxicological profile and physico-chemical properties such as the steam pressure, for example, determine which HFCs are suitable for MDIs. The most promising representatives at present are TG 134a (1,1,2,2-tetrafluoroethane) and TG 227 (1,1,1,2,3,3,3-heptafluoropropane).

For inhalative treatment it may be desirable to have aerosol formulations with two or more active substances. In such cases the active substances are formulated in the necessary concentrations as solutions or suspensions, frequently giving rise to problems regarding the chemical stability of the individual substances or the degree of concentration which can be attained. Major problems are encountered if one of the active substances cannot be suspended or is unstable in a suspension-type formulation of this kind or if one of the active substances is chemically unstable or will not dissolve in a solution-type formulation of this kind, particularly when HFC is used as the propellant.

It is therefore one object of the present invention to develop a formulation for metering aerosols having two or more active substances which overcomes the above-mentioned disadvantages.

### Description of the invention

Surprisingly, it has been found that a plurality of active substances can be formulated as a solution and a suspension combined in one formulation.

The invention relates to stable aerosol preparations with fluorohydrocarbons as propellants, particularly TG 134a and/or TG 227, consisting of two or more active substances, wherein at least one active substance is formulated as a solution by the use of a co-solvent together with at least one active substance formulated as a suspension said active substance formulated as a solution being selected from ipratropium bromide, fenoterol and salts thereof and said active substance formulated as a suspension being selected from salbutamol (albuterol), cromoglycinic acid and salts thereof. The pharmaceutical preparation according to the invention is used for the manufacture of a medicament for treating asthmatic diseases or COPD.

### Detailed description of the invention

A particularly preferred embodiment contains dissolved ipratropium bromide, particularly combined with salbutamol sulphate (albuterol sulphate) as the suspended active substance.

In all the embodiments, the active substances are used in a therapeutically effective quantity, i.e. in a quantity that can induce a successful treatment. The concentration of the active substances and the volume per stroke of spray are adjusted in such a way that the quantity of active substance which is medically necessary or recommended is released by a single spray or by a few sprays.

One embodiment relates to formulations in which the suspended particles are stabilised by the addition of surfactant substances (surfactants) or other suspension-stabilising agents to stabilise the suspended particles against physical changes. The benefit of this is that the particle size will remain pharmaceutically acceptable even over lengthy periods, e.g. during storage. Preferred particle sizes are up to 20 µm, whilst particularly preferred particle sizes are between 5 and 15 µm, best of all not exceeding 10 µm. The advantage of these particle sizes is that the particles are small enough to penetrate deeply into the lungs but not so small as to be breathed out again with the exchanged air.

Suitable surfactants and suspension-stabilising agents include all pharmacologically acceptable substances which have a lipophilic hydrocarbon group and one or more functional hydrophilic groups, especially C₅₋₂₀ fatty alcohols, C₅₋₂₀ fatty acids, C₅₋₂₀ fatty acid esters, lecithin, glycerides, propyleneglycol esters, polyoxyethylenes, polysorbates, sorbitan esters and/or carbohydrates. C₅₋₂₀ fatty acids, propyleneglycol diesters and/or triglycerides and/or sorbitans of the C₅₋₂₀ fatty acids are preferred, whilst oleic acid and sorbitan mono-, di- or trioleates are particularly preferred. Alternatively, toxicologically and pharmaceutically acceptable polymers and block-polymers can be used as suspension-stabilising agents. The surfactants used are either non-fluorinated or partially fluorinated or perfluorinated, the term fluorinated referring to the exchange of hydrogen radicals bound to the carbon for fluorine radicals. The quantity of surfactant may be up to 1:1 based on the proportion by weight of the suspended active substances; amounts of 0.0001:1 to 0.5:1 are preferred, whilst amounts of from 0.0001:1 to 0.25:1 are particularly preferred.

A further advantage of the above surfactants is that they can also be used as valve lubricants. Therefore, one embodiment relates to formulations in which said surfactants are added as valve lubricants.

The presence of the co-solvent has the advantage that the active substance or substances to be dissolved can be formulated in higher concentrations. The addition of co-solvent must not exceed the critical threshold of polarity of the liquid phase at which one of the disadvantages described above begins to affect the suspended particles of active substance.

Suitable co-solvents are pharmacologically acceptable alcohols such as ethanol, esters or water or mixtures thereof; ethanol is preferred. The concentration of the co-solvent in relation to the total formulation may be from 0.0001 to 50 wt.-%, preferably 0.0001 to 25 wt.-%. In another embodiment a concentration of 0.0001 to 10 wt.-% is preferred whilst particularly preferred embodiments are those wherein just enough alcohol is added to dissolve the active substance which has to be dissolved.

In another embodiment, other common propellants are added to the HFC propellant. These added propellants may be, beside other HFCs, saturated lower hydrocarbons such as propane, butane, isobutane or pentane provided that the mixture is pharmacologically acceptable.

In one embodiment, stabilisers are added to the formulation, with a beneficial effect on the pharmaceutical stability of the active substances even over lengthy periods, e.g. during storage. In the context of the invention, stabilisers denotes those substances which prolong the durability and usability of the pharmaceutical preparation by preventing or delaying chemical changes in the individual ingredients, particularly the active substances, e.g. caused by subsequent reactions or degradation, or those which prevent biological contamination. Stabilisers which are preferred for this purpose are those which influence the pH of the liquid phase, such as acids and/or the salts thereof, particularly suitable substances are hydrochloric acid, sulphuric acid, nitric acid, phosphoric acid, ascorbic acid, citric acid and the salts thereof. In addition, preferred bactericides, fungicides etc. are benzalkonium chloride or ethylene diamine tetraacetate. Citric acid is most preferred. The concentration of the stabilisers may be up to 1000 ppm, preferably up to 100 ppm and most preferably 20 to 40 ppm.

One particularly preferred embodiment comprises suspended salbutamol sulphate (albuterol sulphate), dissolved ipratropium bromide, ethanol as co-solvent and citric acid as stabiliser.

### Examples

### Example 1

In a solution of liquefied 89.96 g (1 mol, 89.71 wt.-%) of TG 134a and 10.03 g (218 mmol, 10.00% by weight) of ethanol are dissolved 37 mg (0.09 mmol, 0.037 wt.-%) of ipratropium bromide and 4 mg (20 µmol, 0.004% by weight) of citric acid and 210.5 mg (0.88 mmol, 0.21% by weight) of salbutamol sulphate (albuterol sulphate) are suspended together with 0.05% by weight of surfactant (e.g. 50 mg (177 mmol) of oleic acid).

### Example 2

Analogous to Example 1 using TG 227 as the propellant gas instead of TG 134a.

### Example 3

Disodium chromoglycate is suspended in liquefied P134 and a small amount of ethanol and fenoterol hydrobromide is dissolved therein.

### Example 4

Analogous to Example 3 using TG 227 as propellant gas instead of TG 134a.

## Claims

1. Pharmaceutical preparation for propellant driven metered dose inhalers having a fluorohydrocarbon (HFC) as propellant, which contains a combination of two or more active substances **characterised in that** at least one active substance selected from ipratropium bromide, fenoterol and salts thereof is present in dissolved form by the use of a co-solvent together with at least one other active substance in the form of suspended particles selected from salbutamol (albuterol), cromoglycinic acid and salts thereof.

2. Pharmaceutical preparation according to claim 1, **characterised in that** the combination of active substances consists of two active substances.

3. Pharmaceutical preparation according to claim 1, **characterised in that** the combination of active substances consists of ipratropium bromide and salbutamol sulphate.

4. Pharmaceutical preparation according to any of claims 1 to 3, **characterised in that** the propellant is TG 134a and/or TG 227.

5. Pharmaceutical preparation according to any of claim 1 to 4, **characterised in that** the co-solvent is present in a concentration of 0.0001 to 50% by weight, based on the quantity of liquefied propellant.

6. Pharmaceutical preparation according to any of claim 1 to 4, **characterised in that** the co-solvent is present in a concentration of up to 25% by weight, based on the quantity of liquefied propellant.

7. Pharmaceutical preparation according to any of claim 1 to 4, **characterised in that** the co-solvent is present in a concentration of up to 10% by weight, based on the quantity of liquefied propellant.

8. Pharmaceutical preparation according to any of claim 1 to 7, **characterised in that** the co-solvent is selected from the group of pharmacologically tolerable alcohols, a pharmacologically tolerable ester, water or a mixture thereof.

9. Pharmaceutical preparation according to any of claim 1 to 7, **characterised in that** the co-solvent is ethanol.

10. Pharmaceutical preparation according to any of claims 1 to 9, **characterised in that** the preparation is stabilised by a stabiliser.

11. Pharmaceutical preparation according to claim 10, **characterised in that** the stabiliser contains one or more acids and/or salts.

12. Pharmaceutical preparation according to claim 10, **characterised in that** the stabiliser is selected from the group of hydrochloric acid, sulphuric acid, nitric acid, phosphoric acid, ascorbic acid, citric acid, benzalkonium chloride and/or ethylene diamine tetraacetic acid and/or a salt thereof.

13. Pharmaceutical preparation according to claim 10, **characterised in that** the stabiliser is citric acid.

14. Pharmaceutical preparation according to any of claims 10 to 13, **characterised in that** the stabiliser is present in an amount of up to 100 ppm.

15. Pharmaceutical preparation according to any of claims 10 to 13, **characterised in that** stabiliser is present in an amount of up to 40 ppm.

16. Pharmaceutical preparation according to any of claims 1 to 15, **characterised in that** the preparation contains a surfactant or suspension-stabilising agent.

17. Pharmaceutical preparation according to claim 16, **characterised in that** the surfactant is selected from the group of C₅₋₂₀ fatty alcohols, C₅₋₂₀ fatty acids, C₅₋₂₀ fatty acid esters, lecithin, glycerides, propyleneglycol esters, polyoxyethanes, polysorbates, sorbitan esters and/or carbohydrates.

18. Pharmaceutical preparation according to claim 16, **characterised in that** the surfactant is selected from the group of C₅₋₂₀ fatty acids and/or the esters thereof.

19. Pharmaceutical preparation according to claim 16, **characterised in that** the surfactant is selected from the group of oleic acid and/or sorbitan mono-, di- or trioleate.

20. Pharmaceutical preparation according to claim 16, **characterised in that** the surfactant contains oleic acid.

21. Pharmaceutical preparation according to claim 16 **characterised in that** the surfactant or suspension-stabilising agent comprises a toxicologically acceptable polymer and/or block-polymer.

22. Pharmaceutical preparation according to any of claims 1 to 21 **characterised in that** the preparation comprises ipratropiumbromide, salbutamol sulphate, ethanol, citric acid, TG 227.

23. Pharmaceutical preparation according to any of claims 1 to 21 **characterised in that** the preparation comprises ipratropiumbromide, salbutamol sulphate, ethanol, citric acid, TG 134a.

24. Use of a pharmaceutical preparation according to any claims 1 to 23 for the manufacture of a medicament for treating asthmatic diseases or COPD.

## Patentansprüche

1. Pharmazeutisches Präparat für mit Treibmittel arbeitende Dosierinhalatoren mit einem Fluorkohlenwasserstoff (HFC) als Treibmittel, wobei das Präparat eine Kombination von zwei oder mehr Wirkstoffen enthält, **dadurch gekennzeichnet, dass** mindestens ein Wirkstoff, der unter Ipratropiumbromid, Fenoterol und Salzen davon ausgewählt ist, unter Verwendung eines Co-Lösungsmittels in gelöster Form vorhanden ist zusammen mit mindestens einem weiteren Wirkstoff in Form von suspendierten Teilchen, die unter Salbutamol (Albuterol), Cromoglicinsäure und Salzen davon ausgewählt sind.

2. Pharmazeutisches Präparat nach Anspruch 1, **dadurch gekennzeichnet, dass** die Kombination von Wirkstoffen aus zwei Wirkstoffen besteht.

3. Pharmazeutisches Präparat nach Anspruch 1, **dadurch gekennzeichnet, dass** die Kombination von Wirkstoffen aus Ipratropiumbromid und Salbutamolsulfat besteht.

4. Pharmazeutisches Präparat nach einem der Ansprüche 1 bis 3, **dadurch gekennzeichnet, dass** das Treibmittel TG 134a und/oder TG 227 ist.

5. Pharmazeutisches Präparat nach einem der Ansprüche 1 bis 4, **dadurch gekennzeichnet, dass** das Co-Lösungsmittel in einer Konzentration von 0,0001 bis 50 Gew.-%, bezogen auf die Menge des verflüssigten Treibmittels, vorhanden ist.

6. Pharmazeutisches Präparat nach einem der Ansprüche 1 bis 4, **dadurch gekennzeichnet, dass** das Co-Lösungsmittel in einer Konzentration bis zu 25 Gew.-%, bezogen auf die Menge des verflüssigten Treibmittels, vorhanden ist.

7. Pharmazeutisches Präparat nach einem der Ansprüche 1 bis 4, **dadurch gekennzeichnet, dass** das Co-Lösungsmittel in einer Konzentration bis zu 10 Gew.-%, bezogen auf die Menge des verflüssigten Treibmittels, vorhanden ist.

8. Pharmazeutisches Präparat nach einem der Ansprüche 1 bis 7, **dadurch gekennzeichnet, dass** das Co-Lösungsmittel aus der Gruppe der pharmakologisch verträglichen Alkohole, pharmakologisch verträglichen Ester, Wasser oder Mischungen davon ausgewählt ist.

9. Pharmazeutisches Präparat nach einem der Ansprüche 1 bis 7, **dadurch gekennzeichnet, dass** das Co-Lösungsmittel Ethanol ist.

10. Pharmazeutisches Präparat nach einem der Ansprüche 1 bis 9, **dadurch gekennzeichnet, dass** das Präparat mit einem Stabilisator stabilisiert ist.

11. Pharmazeutisches Präparat nach Anspruch 10, **dadurch gekennzeichnet, dass** der Stabilisator eine oder mehrere Säuren und/oder Salze enthält.

12. Pharmazeutisches Präparat nach Anspruch 10, **dadurch gekennzeichnet, dass** der Stabilisator aus der Gruppe Salzsäure, Schwefelsäure, Salpetersäure, Phosphorsäure, Ascorbinsäure, Citronensäure, Benzalkoniumchlorid und/oder Ethylendiamintetraessigsäure und/oder einem Salz davon ausgewählt ist.

13. Pharmazeutisches Präparat nach Anspruche 10, **dadurch gekennzeichnet, dass** der Stabilisator Citronensäure ist.

14. Pharmazeutisches Präparat nach einem der Ansprüche 10 bis 13, **dadurch gekennzeichnet, dass** der Stabilisator in einer Menge bis zu 100 ppm vorhanden ist.

15. Pharmazeutisches Präparat nach einem der Ansprüche 10 bis 13, **dadurch gekennzeichnet, dass** der Stabilisator in einer Menge bis zu 40 ppm vorhanden ist.

16. Pharmazeutisches Präparat nach einem der Ansprüche 1 bis 15, **dadurch gekennzeichnet, dass** das Präparat ein oberflächenaktives Mittel oder ein suspensionsstabilisierendes Mittel enthält.

17. Pharmazeutisches Präparat nach Anspruch 16, **dadurch gekennzeichnet, dass** das oberflächenaktive Mittel aus der Gruppe C₅₋₂₀-Fettalkohole, C₅₋₂₀-Fettsäuren, C₅₋₂₀-Fettsäureester, Lecithin, Glyceride, Propylenglycolester, Polyoxyethane, Polysorbate, Sorbitanester und/oder Kohlenhydrate ausgewählt ist.

18. Pharmazeutisches Präparat nach Anspruch 16, **dadurch gekennzeichnet, dass** das oberflächenaktive Mittel aus der Gruppe C₅₋₂₀-Fettsäuren und/oder Ester davon ausgewählt ist.

19. Pharmazeutisches Präparat nach Anspruch 16, **dadurch gekennzeichnet, dass** das oberflächenaktive Mittel aus der Gruppe Ölsäure und/oder Sorbitanmono-, -di- oder -trioleat ausgewählt ist.

20. Pharmazeutisches Präparat nach Anspruch 16, **dadurch gekennzeichnet, dass** das oberflächenaktive Mittel Ölsäure enthält.

21. Pharmazeutisches Präparat nach Anspruch 16, **dadurch gekennzeichnet, dass** das oberflächenaktive Mittel oder das suspensionsstabilisierende Mittel ein toxikologisch verträgliches Polymer und/oder Blockpolymer umfasst.

22. Pharmazeutisches Präparat nach einem der Ansprüche 1 bis 21, **dadurch gekennzeichnet, dass** das Präparat ipratropiumbromid, Salbutamolsulfat, Ethanol, Citronensäure und TG 227 umfasst.

23. Pharmazeutisches Präparat nach einem der Ansprüche 1 bis 21, **dadurch gekennzeichnet, dass** das Präparat Ipratropiumbromid, Salbutamolsulfat, Ethanol, Citronensäure und TG 134a umfasst.

24. Verwendung eines pharmazeutischen Präparats nach einem der Ansprüche 1 bis 23 zur Herstellung eines Arzneimittels zur Behandlung von asthmatischen Erkrankungen oder COPD.

## Revendications

1. Préparation pharmaceutique pour inhalateurs à dose mesurée actionnés par propulseur ayant un fluorohydrocarbure (HFC) comme propulseur, qui contient une combinaison de deux ou plusieurs substances actives **caractérisée en ce qu'**au moins une substance active choisie parmi le bromure d'ipratropium, le fénotérol et ses sels est présente sous forme dissoute par l'utilisation d'un cosolvant en même temps qu'au moins une autre substance active sous forme de particules en suspension choisie parmi le salbutamol (albutérol), l'acide chromoglycique et leurs sels.

2. Préparation pharmaceutique selon la revendication 1 **caractérisée en ce que** la combinaison de substances actives consiste en deux substances actives.

3. Préparation pharmaceutique selon la revendication 1 **caractérisée en ce que** la combinaison de substances actives consiste en bromure d'ipratropium et en sulfate de salbutamol.

4. Préparation pharmaceutique selon l'une quelconque des revendications 1 à 3 **caractérisée en ce que** le propulseur est TG 134a et/ou TG 227.

5. Préparation pharmaceutique selon l'une quelconque des revendications 1 à 4 **caractérisée en ce que** le cosolvant est présent en une concentration de 0,0001 à 50 % en masse par rapport à la quantité de propulseur liquéfié.

6. Préparation pharmaceutique selon l'une quelconque des revendications 1 à 4 **caractérisée en ce que** le cosolvant est présent en une concentration pouvant atteindre 25 % en masse par rapport à la quantité de propulseur liquéfié.

7. Préparation pharmaceutique selon l'une quelconque des revendications 1 à 4 **caractérisée en ce que** le cosolvant est présent en une concentration pouvant atteindre 10 % en masse par rapport à la quantité de propulseur liquéfié.

8. Préparation pharmaceutique selon l'une quelconque des revendications 1 à 7 **caractérisée en ce que** le cosolvant est choisi dans le groupe des alcools pharmacologiquement tolérables, d'un ester pharmacologiquement tolérable, de l'eau ou d'un mélange de ceux-ci.

9. Préparation pharmaceutique selon l'une quelconque des revendications 1 à 7 **caractérisée en ce que** le cosolvant est l'éthanol.

10. Préparation pharmaceutique selon l'une quelconque des revendications 1 à 9 **caractérisée en ce que** la préparation est stabilisée par un stabilisant.

11. Préparation pharmaceutique selon la revendication 10 **caractérisée en ce que** le stabilisant contient un ou plusieurs acides et/ou sels.

12. Préparation pharmaceutique selon la revendication 10 **caractérisée en ce que** le stabilisant est choisi dans le groupe de l'acide chlorhydrique, de l'acide sulfurique, de l'acide nitrique, de l'acide phosphorique, de l'acide ascorbique, de l'acide citrique, du chlorure de benzalkonium et/ou de l'acide éthylènediaminetétraacétique et/ou d'un sel de ceux-ci.

13. Préparation pharmaceutique selon la revendication 10 **caractérisée en ce que** le stabilisant est l'acide citrique.

14. Préparation pharmaceutique selon l'une quelconque des revendications 10 à 13 **caractérisée en ce que** le stabilisant est présent en une quantité pouvant atteindre 100 ppm.

15. Préparation pharmaceutique selon l'une quelconque des revendications 10 à 13 **caractérisée en ce que** le stabilisant est présent en une quantité pouvant atteindre 40 ppm.

16. Préparation pharmaceutique selon l'une quelconque des revendications 1 à 15 **caractérisée en ce que** la préparation contient un tensioactif ou agent stabilisant les suspensions.

17. Préparation pharmaceutique selon la revendication 16 **caractérisée en ce que** le tensioactif est choisi dans le groupe des alcools gras en C₅₋₂₀, des acides gras en C₅₋₂₀, des esters d'acides gras en C₅₋₂₀, des lécithines, des glycérides, des esters de propylèneglycol, des polyoxyéthanes, des polysorbates, des esters de sorbitan et/ou des glucides.

18. Préparation pharmaceutique selon la revendication 16 **caractérisée en ce que** le tensioactif est choisi dans le groupe des acides gras en C₅₋₂₀ et/ou de leurs esters.

19. Préparation pharmaceutique selon la revendication 16 **caractérisée en ce que** le tensioactif est choisi dans le groupe de l'acide oléique et/ou du mono-, di- ou trioléate de sorbitan.

20. Préparation pharmaceutique selon la revendication 16 **caractérisée en ce que** le tensioactif contient de l'acide oléique.

21. Préparation pharmaceutique selon la revendication 16 **caractérisée en ce que** le tensioactif ou agent stabilisant les suspensions comprend un polymère et/ou polymère séquencé toxicologiquement acceptable.

22. Préparation pharmaceutique selon l'une quelconque des revendications 1 à 21 **caractérisée en ce que** la préparation comprend du bromure d'ipratropium, du sulfate de salbutamol, de l'éthanol, de l'acide citrique, TG 227.

23. Préparation pharmaceutique selon l'une quelconque des revendications 1 à 21 **caractérisée en ce que** la préparation comprend du bromure d'ipratropium, du sulfate de salbutamol, de l'éthanol, de l'acide citrique, TG 134a.

24. Utilisation d'une préparation pharmaceutique selon l'une quelconque des revendications 1 à 23 pour la fabrication d'un médicament pour traiter les maladies asthmatiques ou la BPCO.
